(19) Europäisches Patentamt
European Patent Office
Office européen des brevets

(11) Veröffentlichungsnummer: **0 131 292**
**A2**

(12) # EUROPÄISCHE PATENTANMELDUNG

(21) Anmeldenummer: **84107952.8**

(22) Anmeldetag: **06.07.84**

(51) Int. Cl.[4]: **C 07 D 249/20**
C 07 D 491/04, G 03 G 5/06
//(C07D491/04, 317/00, 249/00)

(30) Priorität: **08.07.83 DE 3324641**

(43) Veröffentlichungstag der Anmeldung:
**16.01.85 Patentblatt 85/3**

(84) Benannte Vertragsstaaten:
**CH DE FR GB IT LI**

(71) Anmelder: **BASF Aktiengesellschaft**
**Carl-Bosch-Strasse 38**
**D-6700 Ludwigshafen(DE)**

(72) Erfinder: **Neumann, Peter, Dr.**
**Franz-Schubert-Strasse 1**
**D-6908 Wiesloch(DE)**

(72) Erfinder: **Hoffmann, Gerhard, Dr.**
**Pappelstrasse 22**
**D-6701 Otterstadt(DE)**

(54) **Neue Benztriazolverbindungen und deren Verwendung.**

(57) Neue Benztriazolverbindungen der Formel

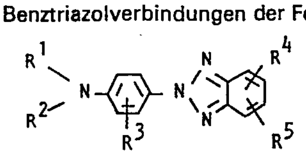

(I),

in der $R^1$ und $R^2$, Alkyl, Alkyl, Phenalkyl oder gegebenenfalls substituiertes Phenyl, $R^3$ H, Alkyl, Alkoxyl oder Halogen und $R^4$ und $R^5$ Halogen, Alkyl, Allyl, Benzyl oder Alkoxy oder – wenn $R^4$ und $R^5$ zueinander orthoständig sind – gemeinsam $-O-(CH_2)_m-O-$ oder $-(CH_2)_n-$ bedeuten, worin m 1, 2 oder 3 und n 3, 4 oder 5 sind und wobei $R^1/R^2$ und/oder $R^4/R^5$ gleich oder verschieden sein können.

Die Triazole I sind als Ladungsträger transportierende Verbindungen in elektrophotographische Aufzeichnungsmaterialien sehr gut geeignet.

EP 0 131 292 A2

Neue Benztriazolverbindungen und deren Verwendung

Die Erfindung betrifft neue Benztriazole und deren Verwendung in elektrophotographischen Aufzeichnungsmaterialien.

Elektrophotographische Verfahren, dafür benötigte Materialien und verschiedene Varianten für den Aufbau von Aufzeichnungsmaterialien sind bekannt. Vorteilhaft für den Einsatz im Reproduktionssektor sind Materialien aus polymeren Bindemitteln, die an spezielle Anforderungen des jeweiligen Einsatzgebietes angepaßt werden können, aus niedermolekularen organischen Verbindungen, die in den Bindemitteln auch in höheren Konzentrationen löslich und die zu einem Transport von Ladungsträgern des elektrischen Stromes befähigt sind, sowie Verbindungen, insbesondere Farbstoffe oder Pigmente, die durch Absorption des bildmäßig eingestrahlten, aktinischen Lichtes Ladungsträger des elektrischen Stromes erzeugen und diese unter Mithilfe des von außen durch die elektrostatische Oberflächenladung aufgeprägten elektrischen Feldes auf die Ladung transportierenden Verbindungen übertragen können. Diese Ladungsträger erzeugenden Verbindungen können je nach dem Einsatzgebiet des Aufzeichnungsmaterials als eigene Schicht innerhalb einer Kompositstruktur eingebracht werden (DE-OS 22 40 408) oder in Form monodispers gelöster Farbstoffmoleküle in der Mischung aus Bindemittel und Ladungsträger transportierenden Verbindungen vorhanden sein (DE-PS 1 058 836). Das in der DE-OS 22 20 408 beschriebene mehrlagige elektrophotographische Aufzeichnungsmaterial besteht aus einem elektrisch leitfähigen Trägermaterial, einer ersten, Farbstoff enthaltenden, etwa 0,005 bis 2 µm dicken, durch Belichtung mit aktinischem Licht Ladungsträger des elektrischen Stromes erzeugenden Schicht aus im Dunkeln isolierenden, organischen Materialien mit mindestens einer Ladungen transportierenden Verbindung.

Es ist auch bekannt, photohalbleitende organische Verbindungen zur Herstellung von elektrophotographischen Druckformen und insbesondere elektrophotographischen Offsetdruckformen zu verwenden (DE-PS 1 117 391 und 1 120 875, DE-AS 15 22 497 und 27 26 116).

Die gestiegenen Anforderungen an Reproduktionssysteme verlangen eine Vielfalt von Aufzeichnungsmaterialien und -systemen, um für spezielle Probleme optimale Lösungen aussuchen zu können. Gewünscht ist eine hohe Lichtempfindlichkeit, eine gute Auflösung und gute Betonerung. Die oft beanstandete ungenügende Betonerung, die auf eine ungünstige Feldstärkedifferenzierung zwischen belichteten und unbelichteten Flächen hinweist, ist hierbei oft auf eine zu hohe Dunkelleitfähigkeit des Aufzeichnungsmaterials im beladenen Zustand zurückzuführen, so daß eine ungenügende

Noe/IG

Oberflächenladungsdichte vor der aktinischen bildmäßigen Belichtung vorliegt.

Der vorliegenden Erfindung lag die Aufgabe zugrunde, elektrophotographische Aufzeichnungsmaterialien insbesondere für die Herstellung von elektrophotographischen Druckformen wie Offsetdruckformen zu entwickeln, die bei hoher Lichtempfindlichkeit, guter Auflösung und Verarbeitung gleichzeitig ein geringes Dunkelleitvermögen aufweisen.

Es wurde gefunden, daß man verbesserte elektrophotographische Aufzeichnungsmaterialien mit elektrisch leitenden Trägern, Ladungsträger erzeugenden Verbindungen bzw. Sensibilisatoren und Ladungsträger transportierenden Verbindungen erhält, wenn diese Materialien als Ladungsträger tranportierende Verbindungen Benztriazole der Formel I enthalten

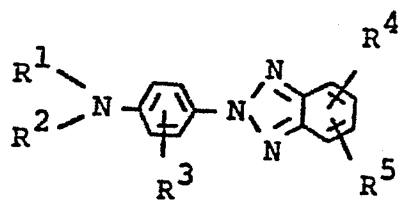

(I),

in der

$R^1$ und $R^2$ unabhängig voneinander für $C_1$- bis $C_6$-Alkyl, Allyl, Phen-$C_1$- bis $C_4$-alkyl oder für gegebenenfalls durch $C_1$- bis $C_4$-Alkyl, $C_1$- bis $C_4$-Alkoxy oder Halogen substituiertes Phenyl,

$R^3$ für Wasserstoff, $C_1$- bis $C_6$-Alkyl, $C_1$- bis $C_6$-Alkoxy oder Halogen,

$R^4$ und $R^5$ unabhängig voneinander für Halogen, $C_1$- bis $C_6$-Alkyl, Allyl, Benzyl, $C_1$- bis $C_6$-Alkoxy oder Benzyloxy oder - wenn $R^4$ und $R^5$ zueinander orthoständig sind - gemeinsam für einen $-O(CH_2)_mO-$ oder einen $-(CH_2)_n$-Rest stehen, worin m 1, 2 oder 3 und n 3, 4 oder 5 sind.

Die elektrophotographischen Aufzeichnungsmaterialien, welche die Benztriazolverbindungen gemäß der vorliegenden Erfindung enthalten, zeichnen sich durch eine Kombination sehr guter Eigenschaften, insbesondere durch eine hohe Photoleitfähigkeit bei gleichzeitig sehr niedriger Dunkelleitfähigkeit aus, so daß die Schichten für die Kopiertechnik sehr geeignet sind. Deutliche Vorteile weisen sie bei der Verwendung für die Herstellung von elektrophotographischen Druckformen auf und genügen hierbei hohen Ansprüchen im Hinblick auf das Auflösungsvermögen und die Druckauflage.

Als Substituenten $R^1$ und $R^2$ kommen außer den bereits bestimmt genannten im einzelnen z.B. in Betracht:

$C_1$- bis $C_6$-Alkyl: Methyl, Ethyl, n- und i-Propyl, n- und i-Butyl, 2-Butyl, n-Pentyl, 2-Pentyl, 3-Methylbutyl-1, 3-Methylbutyl-2, n-Hexyl; Phen-$C_1$- bis $C_4$-alkyl: Benzyl, 1- und 2-Phenylethyl, 1-, 2- und 3-Phenylpropyl, 1-, 2-, 3- und 4-Phenylbutyl;

Gegebenenfalls substituiertes Phenyl: Phenyl, 2-, 3- oder 4-Methylphenyl, 3- oder 4-Ethylphenyl, 3- oder 4-Isopropylphenyl, 4-Butylphenyl, 4-Isobutylphenyl, 4-tert.-Butylphenyl, 2-, 3- oder 4-Methoxyphenyl, 4-Ethoxyphenyl, 4-Propoxyphenyl, 4-Butoxyphenyl, 2-, 3- oder 4-Chlorphenyl, 2-, 3- oder 4-Bromphenyl, 4-Jodphenyl und 4-Fluorphenyl;

Vorzugsweise stehen $R^1$ und $R^2$ für $C_1$- bis $C_4$-Alkyl, wie Methyl, Ethyl, Propyl, Butyl, für Phenyl, oder für durch Methyl, Ethyl, Methoxy oder Ethoxy substituiertes Phenyl und/oder Benzyl.

Für $R^3$ kommen außer Wasserstoff die vorstehend für $R^1$ und $R^2$ genannten Alkylreste und $C_1$- bis $C_6$-Alkoxy, wie Methoxy, Ethoxy, Propoxy, Isopropoxy, Butoxy, Pentoxy und Hexoxy in Betracht. Als Halogen sind z.B. Chlor und Brom zu nennen. Bevorzugt steht $R^3$ für Wasserstoff.

Für $R^4$ und $R^5$ sind im einzelnen zu nennen:

Halogen wie Fluor, Chlor, Brom und Jod;

$C_1$- bis $C_6$-Alkyl: die für $R^1$ und $R^2$ genannten Alkylreste, außerdem Allyl oder Benzyl,

$C_1$- bis $C_6$-Alkoxy: die für $R^3$ genannten Alkoxyreste und Benzyloxy. Wenn $R^4$ und $R^5$ zueinander orthoständig sind, können $R^4$ und $R^5$ gemeinsam ein $-(CH_2)_3-$, $-(CH_2)_4-$, $-(CH_2)_5-$, $-OCH_2O-$, $-OCH_2CH_2O-$ oder $-OCH_2CH_2CH_2O-$-Rest sein.

Vorzugsweise stehen $R^4$ und $R^5$ unabhängig voneinander für Methyl, Ethyl, Methoxy, Ethoxy oder für Methylendioxy und Ethylendioxy.

Besonders bevorzugt sind Benztriazole der Formel II

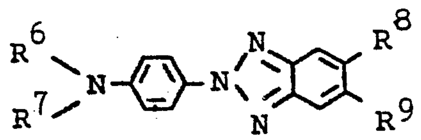

(II),

in der

$R^6$ und $R^7$ für Methyl, Ethyl, Benzyl oder einer der Reste $R^6$ oder $R^7$ für Phenyl oder für durch Methyl, Ethyl, Methoxy oder Ethoxy substituiertes Phenyl,

$R^8$ und $R^9$ für Methyl, Methoxy oder Ethoxy oder $R^8$ und $R^9$ gemeinsam für Methylendioxy oder Ethylendioxy stehen und worin $R^6$ und $R^7$ und/oder $R^8$ und $R^9$ jeweils gleich oder verschieden sein können.

Besonders hervorzuheben sind Benztriazole der Formel II, in der $-N<^{R^6}_{R^7}$ für Diethylamino und $R^8$ und $R^9$ für Methoxy und/oder Ethoxy oder $R^8$ und $R^9$ je für Methoxy und $-N<^{R^6}_{R^7}$ für N-Methylphenylamino, N-Ethyl-benzylamino oder Dibenzylamino stehen.

Die neuen Benztriazole der Formeln I und II können nach bekannten Methoden der organischen Chemie hergestellt werden, beispielsweise aus ortho-Aminoazoverbindungen der Formel

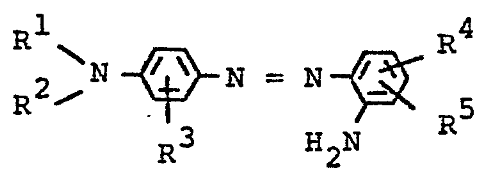 (III)

oder ortho-Nitroazoverbindungen der Formel

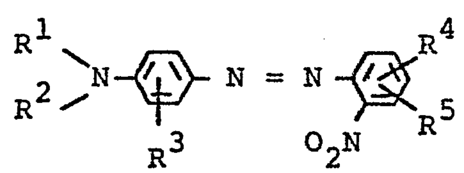 (IV),

in denen $R^1$, $R^2$, $R^3$, $R^4$ und $R^5$ die oben genannten Bedeutungen haben, nach den in Houben-Weyl "Methoden der organischen Chemie", 4. Auflage, Band X/3, S. 425 ff. beschriebenen Methoden. Die Verbindung der Formeln III und IV sind bekannt oder können nach bekannten Verfahren hergestellt werden.

Die Kondensation der Nitroazoverbindungen der Formel IV zu den erfindungsgemäßen Benztriazolen der Formel I erfolgt zweckmäßigerweise in einem Reaktionsschritt. Es kann aber von Vorteil sein, die Umsetzung zweistufig durchzuführen, daß man zunächst die Nitroazoverbindungen der Formel IV zu Benztriazol-N-oxiden (V) oder zu Gemischen von Verbindungen der Formeln I und V umsetzt,

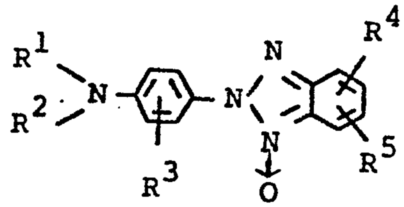

(V)

und dann die Benztriazol-N-oxide in einem zweiten Schritt zu den Benztriazolen der Formel I reduziert, z.B. nach den in Houben-Weyl, "Methoden der organischen Chemie", 4. Auflage, Band 11/1, S. 516 ff, beschriebenen Verfahren.

Die neuen Benztriazole der vorliegenden Erfindung können als bei Belichtung Ladungsträger transportierende Verbindungen sowohl in einschichtig als auch in mehrschichtig auf elektroleitfähige Träger aufgebrachten Aufzeichnungssystemen verwendet werden.

Geeignete einschichtige Systeme weisen bevorzugt auf einem leitfähigen Trägermaterial eine Schicht aus (a) 35 bis 75 Gewichtsteilen eines Bindemittels, (b) 30 bis 60, insbesondere 35 bis 50 Gewichtsteilen der neuen Benzotriazole als Ladungsträger transportierende Verbindungen, (c) bis zu 25 Gewichtsteilen eines weiteren, im wesentlichen inaktiven Bindemittels und (d) 0,05 bis 0,8 Gewichtsteilen einer bei aktinischer Belichtung Ladungsträger erzeugenden Verbindung, insbesondere eines geeigneten Farbstoffs auf. Die Schichten werden vorteilhafterweise aus einer 5 oder 10 gew.%igen Lösung in einem geeigneten organischen Lösungsmittel auf das gereinigte leitfähige Trägermaterial so aufgebracht, daß nach dem Ablüften des Lösungsmittels und Trocknen je nach Verwendungszweck eine Trockenschichtdicke von ca. 0,8 bis 40 µm, z.B. bei elektrophotographischen Druckformen vorzugsweise von 0,8 bis 6 µm resultiert.

Geeignete Mehrschichtsysteme haben auf einem elektroleitfähigen Trägermaterial z.B. (α) eine Ladungsträger erzeugende Schicht und (ß) eine Langstransportschicht aus (ß1) 30 bis 60 Gewichtsteilen mindestens einer Ladungsträger transportierenden Verbindung der Formeln I oder II, (ß2) 15 bis 75 Gewichtsteilen eines organischen Bindemittels und (ß3) bis zu 25 Gewichtsteilen weiterer, die mechanischen Eigenschaften der Schicht verbessernde Zusätze. Die erste Schicht wird vorteilhaft in einer Dicke von 0,005 bis 5, insbesondere von 0,1 bis 0,9 µm aus einer Lösung in einem geeigneten Lösungsmittel auf das Trägermaterial aufgetragen. Nach dem Auftrag der ersten Schicht erfolgt der Auftrag der zweiten Schicht in einer Dicke, daß nach dem Trocknen der Kompositstruktur eine Schichtdicke von 5 bis 25, insbesondere von 7 bis 15 µm resultiert.

Als elektrisch leitende Träger sind prinzipiell alle leitfähigen Trägermaterialien verwendbar, soweit sie für das Anwendungsgebiet geeignet sein. Bevorzugt sind je nach der Anwendung der Aufzeichnungsmaterialien Aluminium-, Zink-, Magnesium-, Kupfer- oder Mehrmetallplatten, z.B. rohe oder vorbehandelte, z.B. aufgerauhte und/oder anodisierte Aluminiumbleche, Aluminiumfolien, Polymerfilme mit metallisierter Oberfläche wie aluminiumbedampfte Polyethylenterephthalatfilme oder auch elektrisch leitende Spezialpapiere. Träger für Druckformen haben vorteilhafterweise eine Dicke von 0,08 bis ca. 0,3 mm.

Zwischen dem Träger und den oben genannten Schichten kann eine weitere Schicht mit einer Dicke von 0,03 bis 5 µm zur Verbesserung der Haftung und/oder der elektrophotographischen Eigenschaften angeordnet werden.

Die Art der geeigneten organischen Bindemittel für die Schichten richtet sich nach dem beabsichtigten Verwendungszweck der Aufzeichnungsmaterialien. Für den Kopiersektor eignen sich z.B. Celluloseether, Polyesterharze, Polyvinylchloride, Polycarbonate, Copolymere, wie Styrol-Maleinsäureanhydrid-Copolymere oder Vinylchlorid-Maleinsäureanhydrid-Copolymere oder Mischungen solcher Bindemittel. Bei ihrer Auswahl spielen ihre filmbildenden und elektrischen Eigenschaften, ihre Haftfestigkeit auf dem Trägermaterial und ihre Löslichkeitseigenschaften eine besondere Rolle. Insbesondere bei Aufzeichnungsmaterialien für die Herstellung elektrophotographischer Druckplatten und besonders bei denen für den Offsetdruck sind solche besonders geeignet, die in basischen wäßrigen oder alkoholischen Lösungsmitteln löslich sind. Dies sind vor allem Substanzen mit alkalilöslich machenden Gruppen wie Anhydrid-, Carboxyl-, Sulfonsäure-, Phenol- oder Sulfonimid-Gruppierungen. Bevorzugt sind Bindemittel, insbesondere solche mit hohen Säurezahlen, die in basischen wäßrig-alkoholischen Lösungsmittelsystemen leicht löslich sind und ein mittlerer Molekulargewicht (Gewichtsmittel) von 800 bis 50 000 und insbesondere von 1 500 bis 10 000 aufweisen. Geeignet sind z.B. Copolymerisate aus Methacrylsäure und Methacrylsäureestern, besonders Copolymerisate aus Styrol und Maleinsäureanhydrid und aus Styrol, Methacrylsäure und Methacrylsäureester, soweit sie die vorstehende Löslichkeitsbedingung erfüllen. Obwohl bekanntermaßen Bindemittel mit freien Carboxylgruppen die Dunkelleitfähigkeit der elektrophotographischen Schichten in unerwünschter Weise erhöhen und dadurch zu schlechten Betonerungsergebnissen führen, lassen sich solche Bindemittel leicht an die neuen Benztriazole anpassen. So hat sich gezeigt, daß Copolymerisate aus Styrol, Maleinsäureanhydrid und Acryl- oder Methacrylsäure, die einen Anteil von einpolymerisiertem Maleinsäureanhydrid von 5 bis 50 Gew.% und einen Anteil von einpolymerisierter Acryl- oder Methacrylsäure von 5 bis 35, insbesondere 10 bis 30 Gew.%

aufweisen, befriedigende elektrophotographische Schichten mit ausreichender Dunkelleitfähigkeit liefern. Sie weisen eine hervorragende Löslichkeit in Auswaschmitteln aus 75 Gew.% Wasser, 23 Gew.% Isobutanol und 2 Gew.% Soda auf, sind jedoch in offsettypischem Wischwasser unlöslich.

Geeignete Ladungsträger erzeugende Verbindungen bzw. Sensibilisatoren sind für einschichtig aufgetragene Systeme, wie sie auch zur Herstellung elektrophotographischer Druckformen dienen, z.B. Farbstoffe aus der Triarylmethanreihe, Xanthenfarbstoffe und Cyaninfarbstoffe. Sehr gute Ergebnisse wurden mit den neuen Verbindungen der Formel I zusammen mit Rhodamin B (C.I. 45 170), Rhodamin 6 G (C.I. 45 160), Malachitgrün (C.I. Basic Green 4; C.I. 42 000), Methylviolett (C.I. 42 535) oder Kristallviolett (C.I. 42 555) erhalten. Bei mehrschichtig aufgetragenen Systemen liegt der Farbstoff oder das Pigment in einer separaten Ladungsträger erzeugenden Schicht vor. Hier sind Azofarbstoffe, Phthalocyanine, Isoindolinfarbstoffe und Perylentetracarbonsäurederivate besonders wirksam. Gute Ergebnisse werden mit Perylen-3,4,9,10-tetracarbonsäurediimidderivaten erzielt, wie sie in den DE-OS 31 10 954 und 31 10 960 beschrieben sind.

Für die jeweilige Verwendung kann das mit den neuen Benztriazolen hergestellte elektrophotographische Aufzeichnungsmaterial noch die üblichen Zusätze enthalten, z.B. Verlaufmittel und Weichmacher in der photoleitfähigen Schicht oder monodispers gelöste Haftvermittler zur Verbesserung der Haftung zwischen Träger und Schicht oder zwischen den Schichten bei mehrlagigen Systemen.

Die mit den erfindungsgemäßen Benztriazolen erhaltenen elektrophotographischen Aufzeichnungsmaterialien zeichnen sich durch eine Kombination sehr guter Eigenschaften, insbesondere einer hohen Photoleitfähigkeit bei gleichzeitig sehr niedriger Dunkelleitfähigkeit aus, so daß die Schichten für die Kopiertechnik sehr geeignet sind.

Deutliche Vorteile weisen diese Materialien bei der Verwendung für die Herstellung von elektrophotographischen Druckformen auf und genügen hierbei hohen Anforderungen im Hinblick auf das Auflösevermögen und die Druckauflage. Die hohe Lichtempfindlichkeit erlaubt eine Senkung der Belichtungszeit bei der Verarbeitung in der Reprokamera gegenüber handelsüblichen Materialien bis etwa um die Hälfte. Aus einer sehr randscharfen Bildwiedergabe resultiert eine gute Auflösung. Auch feine Rasterpunkte in den lichten Tonwortbereichen können gut wiedergegeben werden. Ferner führt die Belichtung der Schichten zu sehr geringen Restspannungen und die bei der Betonerung erhaltenen Bilder zeichnen sich durch gute Grundfreiheit in den Nichtbildbereichen aus. Die spektrale Empfindlichkeit sinkt bei 600 nm stark ab, so daß die Schichten bei Rotlicht gehandhabt werden können, ohne daß Bildverluste auftreten.

Die Herstellung elektrophotographischer Offsetdruckformen erfolgt wie üblich durch eine elektrostatische Aufladung des elektrophotographischen Aufzeichnungsmaterials mittels einer Hochspannungscorona, eine direkt nachfolgende bildmäßige Belichtung, die Entwicklung des vorliegenden elektrostatischen, latenten Ladungsbildes mittels eines Trocken- oder Flüssigtoners, die Fixierung des Toners durch einen nachfolgenden Schmelzvorgang und die Entfernung der unbetonerten, photohalbleitenden Schicht mittels eines geeigneten Auswaschmittels. Die so erhaltene Druckform kann in bekannter Weise für den Offsetdruck noch vorbereitet werden, z.B. durch eine Hydrophilierung und Gummierung der wasserführenden Oberfläche.

Die folgenden Anwendungsbeispiele sollen die neuen Benzotriazole und deren Anwendung zusätzlich erläutern und belegen.

Die genannten Teile und Prozentangaben beziehen sich auf das Gewicht.

I. Herstellung der Benztriazole

Beispiel 1

a) 5,94 g 2-Nitro-4,5-dimethoxyanilin (hergestellt nach Synthesis 1978, 924) werden in 40 ml Eisessig und 9,12 g konz. Salzsäure bei 0 bis 10°C mit einer Lösung von 2,3 g Natriumnitrit in 5 ml Wasser diazotiert. Man rührt noch 2 Stunden bei 0 bis 10°C nach und filtriert die Lösung des Diazoniumsalzes nach Zusatz einer Spatelspitze Amidosulfonsäure in ein Gemisch aus 4,3 g N,N-Diethylanilin, 50 ml Wasser und 20 g Natriumacetat. Durch Zusatz von weiterem Natriumacetat wird der pH auf ca. 5 eingestellt. Man rührt 12 Stunden bei Raumtemperatur nach und isoliert und trocknet den Azofarbstoff in üblicher Weise. Ausbeute 10,3 g.

b) 8 g der Azoverbindung werden in 30 ml Triethylphosphit 4 Stunden zum Sieden erhitzt. Anschließend engt man unter vermindertem Druck ein, verrührt den warmen Rückstand mit 20 ml Isopropanol, saugt den Niederschlag ab, wäscht ihn mit Hexan und trocknet. Nach Umkristallisation aus Isopropanol erhält man 3,3 g des Benztriazols der Formel

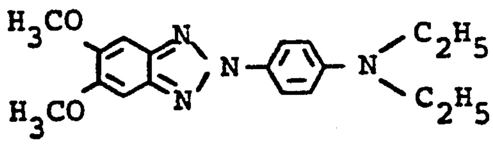

Schmp. 159 bis 160°C.

Analyse: $C_{18}H_{22}N_4O_2$ (326)
ber. C 66,24 H 6,79 N 17,16 O 9,80
gef. C 66,5 H 6,9 N 17,3 O 9,9

Beispiel 2

a) 8,5 g eines Isomerengemisches aus 2-Nitro-4-methoxy-5-ethoxy- und 2-Nitro-4-ethoxy-5-methoxyanilin (hergestellt analog Synthesis 1978, 924 aus 1-Methoxy-2-ethoxybenzol) werden analog Beispiel 1a) diazotiert und auf 6 g N,N-Diethylanilin gekuppelt. Man erhält 13,5 g der Azofarbstoffmischung.

b) 13 g dieses Gemisches werden in 150 ml Triethylphosphit analog Beispiel 1b) umgesetzt. Nach Umkristallisation aus Isopropanol erhält man 6 g des Benztriazols der Formel

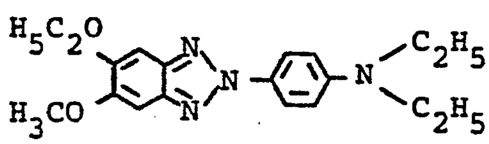

Schmp. 128 bis 129°C.

Analyse: $C_{19}H_{24}N_4O_2$ (340)
ber. C 67,04 H 7,11 N 16,46 O 9,40
gef. C 66,7 H 6,8 N 16,3 O 9,5

Beispiel 3

a) 7,2 g 2-Nitro-4,5-methylendioxyanilin (hergestellt nach Synthesis 1978, 924) werden analog Beispiel 1a) diazotiert und auf 6 g N,N-Diethylanilin gekuppelt. Man erhält 10,3 g Azofarbstoff.

b) 10,0 g Azoverbindung werden in 100 ml Triethylphosphit analog Beispiel 1b) umgesetzt. Nach Umkristallisation aus Isopropanol erhält man 1,8 g des Benztriazols der Formel

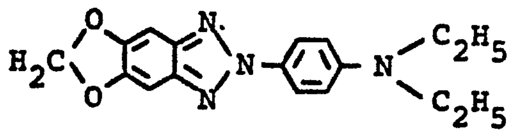

Schmp. 185 bis 187°C.

Analyse: $C_{17}H_{18}N_4O_2$ (310)

| | | | | |
|---|---|---|---|---|
| ber. | C 65,79 | H 5,85 | N 18,05 | O 10,31 |
| gef. | C 66,0 | H 5,8 | N 17,6 | O 10,6 |

Beispiel 4

a) 9,9 g 2-Nitro-4,5-dimethoxyanilin werden analog Beispiel 1a) diazotiert und auf 9,15 g N-Methyldiphenylamin gekuppelt. Man erhält 20,2 g des Azofarbstoffs.

b) 20 g der Azoverbindung werden in 100 ml Triethylphosphit analog Beispiel 1b) umgesetzt. Nach Umkristallisation erhält man 4,4 g des Benztriazols der Formel

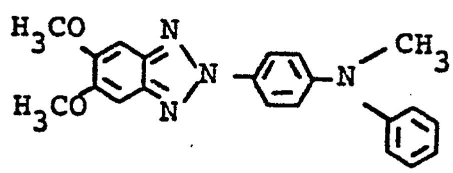

Schmp. 144 bis 145°C (aus Isopropanol).

Analyse:

| | | | | |
|---|---|---|---|---|
| ber. | C 69,98 | H 5,59 | N 15,54 | O 8,88 |
| gef. | C 69,7 | H 5,6 | N 15,4 | O 9,3 |

Beispiel 5

a) 14,6 g 2-Nitro-4,5-methylendioxyanilin werden analog Beispiel 1a) diazotiert und auf 14,7 g N-Methyldiphenylamin gekuppelt. Man erhält 22,7 g Azofarbstoff.

b) 20,7 g der Azoverbindung werden in 150 ml Triethylphosphit analog Beispiel 1b) umgesetzt. Nach Umkristalisation aus Ethylglykol erhält man 9 g des Benztriazols der Formel

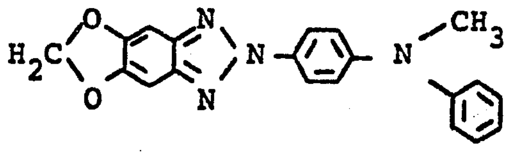

Schmp. 205 bis 206°C.

Analyse: $C_{20}H_{16}N_4O_2$ (344)
ber. C 69,76 H 4,68 N 16,27 O 9,29
gef. C 69,5 H 4,6 N 16,1 O 9,5

Beispiel 6

a) 14,9 g 2-Nitro-4,5-dimethoxyanilin werden analog Beispiel 1a) diazotiert und auf 15,8 g N-Ethyl-N-benzylanilin gekuppelt. Man erhält 28,1 g Azofarbstoff.

b) 21 g der Azoverbindung werden in 120 ml Triethylphosphit analog Beispiel 1b) umgesetzt. Nach Umkristallisation aus Isobutanol und Säulenchromatographie (Kieselgel/Methylenchlorid) erhält man 3,4 g des analysenreinen Benztriazols der Formel

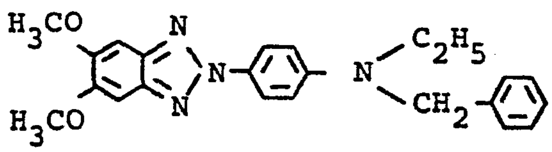

Schmp. 118 bis 119°C.

Analyse: $C_{23}H_{24}N_4O_2$ (388)
ber. C 71,11 H 6,23 N 14,42 O 8,24
gef. C 71,0 H 6,3 N 14,8 O 8,3

Beispiel 7

a) 14,9 g 2-Nitro-4,5-dimethoxyanilin werden analog Beispiel 1a) diazotiert und auf 20,5 g N,N-Dibenzylanilin gekuppel. Man erhält 30,8 g Azofarbstoff.

b) 24,1 g der Azoverbindung werden in 120 ml Triethylphosphit analog Beispiel 1b) umgesetzt. Nach Umkristallisation aus Isobutanol erhält man 6,4 g des Benztriazols der Formel

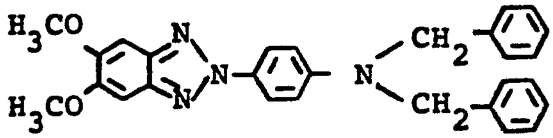

Schmp. 175 bis 177°C.

Analyse: $C_{28}H_{26}N_4O_2$ (450)

| | | | | |
|---|---|---|---|---|
| ber. | C 74,65 | H 5,82 | N 12,44 | O 7,10 |
| gef. | C 74,4 | H 5,8 | N 12,7 | O 7,5 |

Beispiel 8

a) 14,6 g 2-Nitro-4,5-methylendioxyanilin werden analog Beispiel 1a) diazotiert und auf 16,9 g N-Ethyl-N-benzylanilin gekuppelt. Man erhält 24,2 g Azofarbstoff.

b) 22,2 g der Azoverbindung werden in 150 ml Triethylphosphit analog Beispiel 1b) umgesetzt. Nach Umkristallisation aus Ethylglykol erhält man 8,9 g des Benztriazols der Formel

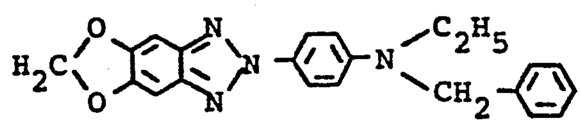

Schmp. 200 bis 201°C.

Analyse: $C_{22}H_{20}N_4O_2$ (372)

| | | | | |
|---|---|---|---|---|
| ber. | C 70,95 | H 5,41 | N 15,04 | O 8,59 |
| gef. | C 70,7 | H 5,3 | N 14,8 | O 9,1 |

Beispiel 9

a) 6,85 g 4,5-Dimethyl-2-nitroanilin (97 %ig) werden analog Beispiel 1a) diazotiert und auf 6 g N,N-Diethylanilin gekuppelt. Man erhält 13 g Azofarbstoff.

b) 10 g der Azoverbindung werden in 60 ml Triethylphosphit analog Beispiel 1b) umgesetzt. Nach Umkristallisation aus Ethanol erhält man 5,1 g des Benztriazols der Formel

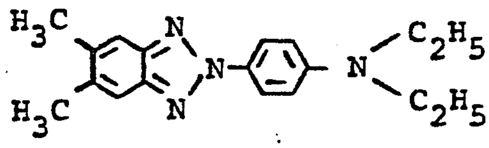

Schmp. 160 bis 162°C.

Analyse: $C_{18}H_{22}N_4$ (294)
ber. C 73,44 H 7,53 N 19,03
gef. C 73,4 H 7,5 N 18,9

II. Anwendung der Benztriazole (I)

Anwendungsbeispiel 1

Die in den Beispielen 1 bis 8 beschriebenen Verbindungen wurden nach einer einheitlichen Rezeptur zu homogenen mit dem Farbstoff Rhodamin B (C.I. 45 170) sensibilisierten Photoleiterschichten verarbeitet. Die folgende Rezeptur wurde gewählt:

| | | |
|---|---|---|
| Bindemittel | 55 | Gewichtsteile |
| Benztriazol Formel I | 45 | Gewichtsteile |
| Rhodamin B | 0,6 | Gewichtsteile |
| Tetrahydrofuran | 700 | Gewichtsteile |
| Methylglykol | 200 | Gewichtsteile |

Als Bindemittel diente ein Copolymerisat aus Styrol, Methacrylsäure und Maleinsäureanhydrid im Verhältnis 55 : 30 : 15 mit einem mittleren Molekulargewicht von etwa 20 000.

Die Lösung wurde auf ein fein gebürstetes Aluminiumblech (Dicke 0,1 mm) mit einer Rakel in einer solchen Naßschichtdicke aufgebracht, daß nach dem Verdampfen der Lösemittel und einer Trocknung der Schichten bei 80°C (Dauer: 15 Minuten) eine Trockenschichtdicke von etwa 4 $\pm$ 0,2 µm verblieb. Diese Schichten wurden dann 48 Stunden bei Raumtemperatur und einer relativen Luftfeuchte von 60 % im Dunkeln gelagert.

Zuerst wurden die Schichten mit einer Gleichspannungscorona von -8,5 kV im Abstand von 10 mm 20 Sekunden lang beladen.

Danach wurde das erreichte Oberflächenpotential (Meßgröße a) ermittelt. Die so beladenen Schichten verblieben für weitere 20 Sekunden im Dunkeln. Danach wurde das noch vorhandene Oberflächenpotential ermittelt und daraus der eingetretene Potentialabfall in Prozent, bezogen auf das Ausgangspotential, errechnet (Meßgröße b).

Dann wurde jeweils eine Sekunde lang mit dem weißen Licht einer Xenonhochdrucklampe (Leistungsaufnahme: 150 W) belichtet und das Oberflächenpotential bestimmt. Aus dem Oberflächenpotential vor und nach der Belichtung wurde der photoinduzierte Potentialabfall in Prozent, bezogen auf das Potential direkt vor der Belichtung, errechnet (Meßgröße c).

Weiterhin wurden die Schichten dann mit einer Gleichspannungscorona von -8,0 kV in 1 cm Abstand auf ein Oberflächenpotential von 500 Volt beladen und mit dem Licht einer Xenonlampe (Leistungsaufnahme: 150 Watt), das mittels eines Neutralfilters mit einer optischen Dichte 1,0 und einem Interferenzfilter ($\lambda_{max}$ = 551 nm) mit einem Durchlässigkeitsfaktor von 0,45 abgeschwächt worden war, belichtet. Die gemessene Beleuchtungsstärke in Schichtebene betrug etwa 3,5 $\mu W.cm^2$. Gemessen wurde die Zeit bis zum Abfall des ursprünglich vorhandenen Potentials auf die Hälfte (Halbwertszeit). Das Produkt aus dieser Halbwertszeit und der Beleuchtungsstärke ist die Halbwertsphotoempfindlichkeit in $\mu J.cm^2$ (Meßgröße d).

Weiterhin wurden die Schichten auf Auskristallisation bzw. Homogenität geprüft. Außerdem wurden nach bekannten Methoden die Erweichungsbereiche ermittelt. Die Ergebnisse sind in der Tabelle 1 zusammengestellt.

Tabelle 1

| Anwendungs-beispiel | Verbindung aus Bei-spiel | verträglich mit Binde-mittel | Meßgröße a [V] | b [%] | c [%] | d [µJ.cm$^{-2}$] | Schichter-weichungs-bereich [°C] |
|---|---|---|---|---|---|---|---|
| 1.1 | 1 | ja | 1360 | 18,5 | 83,0 | 29,0 | 140 - 145 |
| 1.2 | 2 | ja | 1300 | 23,0 | 76,5 | 26,0 | 130 - 135 |
| 1.3 | 4 | ja | 1070 | 13,0 | 82,5 | 25,0 | 135 - 140 |
| 1.4 | 6 | ja | 1410 | 12,0 | 89,1 | 24,0 | 135 - 140 |
| 1.5 | 7 | ja | 1330 | 24,5 | 79,5 | 33 | 140 - 145 |

Meßgröße a: Oberflächenpotential nach Beladen mit einer Gleichspannungscorona von −8,5 kV; Abstand 10 mm; Dauer: 20 s

Meßgröße b: Dunkelabfall in %, bezogen auf Meßgröße a

Meßgröße c: photoinduzierter Potentialabfall in %, bezogen auf Potential vor der Belichtung

Meßgröße d: Halbwertsphotoempfindlichkeit

Wie die Tabelle 1 zeigt, weisen die erfindungsgemäßen Verbindungen die gewünschte hohe Photoempfindlichkeit auf, sind mit dem Bindemittel ausgezeichnet verträglich und verleihen den Photoleiterschichten einen praktikablen Erweichungsbereich.

Anwendungsbeispiel 2

Auf eine Polyesterfolie mit einer aufgedampften, leitfähigen Aluminiumschicht in einer Dicke von etwa 300 Å wird eine Schicht aus 60 Gewichtsteilen eines chlorierten Perylen-3,4:9,10-tetracarbonsäurediimidbisbenzimidazols mit einem Chlorgehalt von etwa 38 Gew.% und 50 Gewichtsteilen eines Copolymerisates aus Vinylchlorid, Acrylsäure und einem Maleinsäurediester in einer Dicke von etwa 0,55 µm aufgebracht.

Auf diese Ladungsträger erzeugende Schicht wird aus einer Lösung in Essigsäureethylester eine Ladungstransportschicht aus 45 Gewichtsteilen eines Polycarbonats mit einer Schmelztemperatur von 220 bis 230°C, 10 Gewichtsteilen eines Polyesters mit einer Säurezahl von etwa 40 und einem mittleren Molekulargewicht von etwa 4500 und 40 Gewichtsteilen der Verbindung aus Beispiel 7 so aufgebracht, daß die Trockenschichtdicke 8 µm beträgt. Nach dem Ablüften der Lösemittel werden die Schichten für 30 Minuten bei 80°C getrocknet.

Die Schicht wurde dann mit einer Gleichspannungscorona von -7,5 kV in 1 cm Abstand gleichmäßig auf ein Oberflächenpotential von 600 Volt beladen und dann mit dem weißen Licht einer Xenonlampe mit einer Beleuchtungsstärke von etwa 60 $\mu W.cm^{-2}$ belichtet. Es wurde die Zeit ermittelt, in der das Oberflächenpotential auf die Hälfte (300 Volt) sinkt (Halbwertsbelichtungszeit): 65 Millisekunden. Parallel hierzu wurde über die gleiche Zeit an einer gleich behandelten nicht belichteten Schicht der Potentialabfall gemessen, der im Dunkeln als Folge der Dunkelleitfähigkeit der eintritt; dieser Potentialabfall im Dunkeln beträgt innerhalb der Halbwertsbelichtungszeit etwa 0,2 Volt.

Anwendungsbeispiel 3

Eine Lösung aus 45 g der nach Beispiel 1 erhaltenen Verbindung, 10 g eines chlorierten Polyvinylchlorids, 50 g eines Copolymerisats aus 55 Gewichtsteilen Styrol, 40 Gewichtsteilen Maleinsäureanhydrid und 5 Gewichtsteilen Methacrylsäure und 0,3 Gewichtsteilen Malachitgrün in 950 g Tetrahydrofuran wird auf eine durch Drahtbürstung mechanisch aufgerauhte 100 µm starke Aluminiumfolie mit einer Bürsttiefe von etwa 3 µ aufgetragen. Nach dem Verdunsten des Lösungsmittels hinterbleibt eine Photoleiterschicht mit einer Dicke von 4 bis 5 µm. Die Schicht wird in der bei der Elektrophotographie üblichen Weise mit einer Corona auf ein Oberflächenpotential von -600 V aufgeladen und bildmäßig mit einer Xenonhochdrucklampe von 2 kW Leistung für 50 Sekunden belichtet. Das entstandene elektrostatische Bild der Vorlage wird durch Einstäuben mit einem durch Ruß angefärbten Harzpulver sichtbar gemacht und durch Erwärmen auf 120°C zu einer wischfesten Elektrokopie fixiert. Man erhält so eine der Vorlage entsprechende Elektrokopie, welche an den Bildstellen gegen alkalische Lösungen beständig ist.

Zur Umwandlung der so hergestellten Elektrokopie in eine Flachdruckform wird die Kopie mit einer Lösung behandelt, die aus 75 Gew.% Wasser, 20 Gew.% Isopropanol, 4,5 Gew.% Natriumsilikat und 0,5 Gew.% Soda besteht. Nach kurzem Spülen mit Wasser und Einfärben mit fetter Farbe können von der Druckform Abdrücke hergestellt werden.

Anwendungsbeispiele 4 und 5

Man verfährt wie im Anwendungsbeispiel 3, jedoch verwendet man anstelle der dort angegebenen Triazolverbindung die der Beispiele 4 bzw. 6. Man erhält praktisch die gleichen Ergebnisse, nur daß die Belichtungszeit im Falle des Triazols aus Beispiel 4 auf 55 Sekunden und im Falle des Triazols aus Beispiel 6 auf 35 Sekunden reduziert werden müssen.

Hk.

Patentansprüche

1. Neue Benztriazolverbindungen der Formel

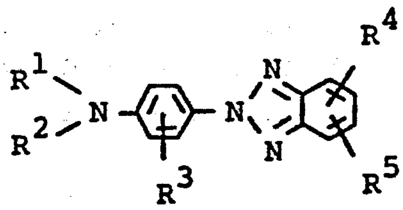

(I),

in der

$R^1$ und $R^2$ unabhängig voneinander für $C_1$- bis $C_6$-Alkyl, Allyl, Phen-$C_1$- bis $C_4$-alkyl oder für gegebenenfalls durch $C_1$- bis $C_4$-Alkyl, $C_1$- bis $C_4$-Alkoxy und/oder Halogen substituiertes Phenyl,

$R^3$ für Wasserstoff, $C_1$- bis $C_6$-Alkyl, $C_1$- bis $C_6$-Alkoxy oder Halogen,

$R^4$ und $R^5$ unabhängig voneinander für Halogen, $C_1$- bis $C_6$-Alkyl, Allyl, Benzyl, $C_1$- bis $C_6$-Alkoxy oder Benzyloxy oder - wenn $R^4$ und $R^5$ zueinander orthoständig sind - gemeinsam für einen $-O-(CH_2)_mO-$ oder einen $-(CH_2)_n$-Rest stehen, worin

m 1, 2 oder 3 und

n 3, 4 oder 5 sind.

2. Benztriazolverbindungen gemäß Anspruch 1, dadurch gekennzeichnet, daß $R^1$ und $R^2$ für $C_1$- bis $C_4$-Alkyl, für gegebenenfalls durch Methyl, Ethyl, Methoxy oder Ethoxy substituiertes Phenyl und/oder Benzyl stehen.

3. Benztriazolverbindungen gemäß Anspruch 1 oder 2, dadurch gekennzeichnet, daß $R^3$ für Wasserstoff steht.

4. Benztriazolverbindungen gemäß Anspruch 1, 2 oder 3, dadurch gekennzeichnet, daß $R^4$ und $R^5$ unabhängig voneinander für Methyl, Ethyl, Methoxy, Ethoxy oder für Methylendioxy oder Ethylendioxy stehen.

5. Benztriazolverbindungen gemäß Anspruch 1, gekennzeichnet durch die Formel

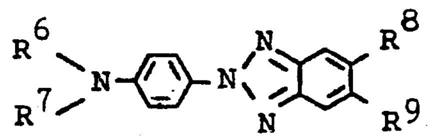 (II),

in der
$R^6$ und $R^7$ für Methyl, Ethyl, Benzyl oder einer der Reste $R^6$ oder $R^7$ für Phenyl oder für durch Methyl, Ethyl, Methoxy oder Ethoxy substituiertes Phenyl und
$R^8$ und $R^9$ für Methyl, Methoxy, Ethoxy oder $R^8$ und $R^9$ gemeinsam für Methylendioxy oder Ethylendioxy stehen, wobei
$R^6$, $R^7$, $R^8$ und $R^9$ jeweils gleich oder verschieden sein können.

6. Benztriazolverbindungen gemäß Anspruch 5, dadurch gekennzeichnet, daß in der Formel

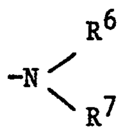

für Diethylamino und $R^8$ und $R^9$ je für Methoxy oder Ethoxy oder $R^8$ und $R^9$ für Methoxy und

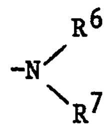

für N-Methylphenylamino, N-Ethylbenzylamino oder Dibenzylamino stehen.

7. Verwendung der Benztriazolverbindungen gemäß den Ansprüchen 1, 2, 3, 4, 5 oder 6 zur Herstellung von elektrophotographischen Aufzeichnungsmaterialien und Druckformen.